# EUROPEAN PATENT SPECIFICATION

(11) **EP 0 572 599 B1**
(45) Date of publication and mention of the grant of the patent: **17.06.1998**
(21) Application number: 92924725.2
(22) Date of filing: 14.12.1992
(51) Int. Cl.: G01N 33/84, G01N 31/22, G01N 33/543, C09B 29/36, C09B 33/12, C07D 323/00

(54) **ION-SENSITIVE DYES**
ION-SENSIBLE FARBSTOFFE
COLORANTS SENSIBLES AUX IONS

(30) Priority: 18.12.1991 GB 9126829
(43) Date of publication of application: 08.12.1993
(73) Proprietor: KODAK LIMITED, Harrow, Middlesex HA1 4TY (GB); Johnson & Johnson Clinical Diagnostics, Inc., Rochester New York 14650 (US)
(72) Inventor: MOORE, Christopher Peter, Harrow, Middx HA2 6HP (GB); SUTHERLAND, The Chase, Wirral L48 2JD (GB); KING, Angela, Netherton Bootle, Merseyside L30 7PH (GB)
(74) Representative: Mercer, Christopher Paul
(86) International application number: EP9202888
(87) International publication number: WO9312428

(56) References cited:
- EP-A- 0 287 326
- US-A- 4 742 010
- JOURNAL OF THE CHEMICAL SOCIETY, PERKIN TRANSACTIONS 1 no. 1, 1989, LETCHWORTH GB pages 195 - 196 S.SHINKAI ET AL. 'autoaccelerative diazo coupling with calixÄ4Üarene: unusual co-operativity of the calixarene hydroxy groups'
- CHEMISTRY LETTERS no. 7, 1989, TOKYO JP pages 1125 - 1126 E. NOMURA ET AL. 'selective ion extraction by a calixÄ6Üarene derivative containing azo groups'
- CHEMICAL ABSTRACTS, vol. 107, no. 20, 16 November 1987, Columbus, Ohio, US; abstract no. 189997x, T.SHONO '14-crown-4 ethers containing phenylazo groups for metal-ion extraction photometric reagents and as ionophores' page 957 ;

## Description

The invention relates to compounds which are ion-sensitive dyes- More particularly, the invention relates to dyes which form complexes with cations.

Compounds known as chromoionophores in which an ionophore is directly linked to a chromophore are known. The interaction of such chromoionophores with an ion can result in a colour change. In some cases, the chromoionophore will interact selectively with a particular ion in preference to other ions.

Chromoionophores in which an azo dye chromogenic group is attached to a crown ether ionophore are described in J.F. Alder, D.C. Ashworth, R. Narayanaswamy, R.E. Moss and I.O. Sutherland, Analyst, 1987, 112, 1191. An optical fibre probe utilising such a chromoionophore is reported to respond to potassium ions in aqueous solution with a K⁺/Na⁺ selectivity ratio of 6.4.

There is a need for alternative cation-sensitive chromoionophores which give rise to significantly modified visible spectra upon interaction with cations. There is a particular need for such chromoionophores which can interact selectively with cations and which preferably can provide greater selectivity than known chromoionophores.

In a particular application, suitable chromoionophores are required for sensors for the determination of metal ions of physiological importance, for example, the measurement of potassium ions in mammalian blood in the presence of other metal ions. For such an application, the chromoionophore should preferably be highly selective, capable of functioning at physiological pH and capable of responding to the metal ion in the range of concentration in which it is generally present.

The invention provides chromoionophores in the form of ion-sensitive dyes which meet one or more of the above-identified requirements.

The compounds of the invention are represented by the formula 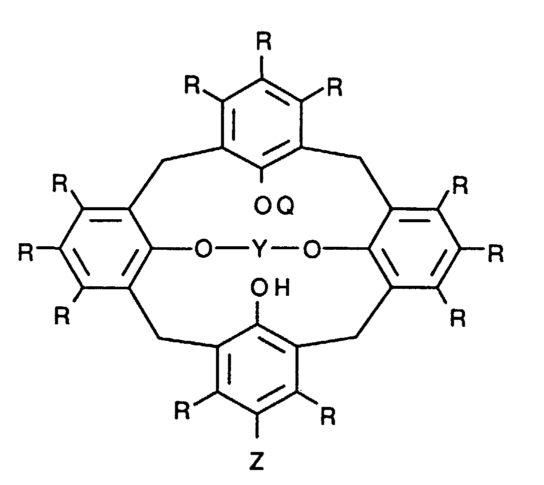 wherein
Y is -(CH₂)₂[O(CH₂)₂]ₙ- in which n is an integer from 1 to 6;
Z is an azo dye group, -N=N-Ar, in which Ar is a substituted or unsubstituted aryl group;
Q is hydrogen or an alkyl group having from 1 to 4 carbon atoms;
each R independently is hydrogen or a substituent free of acidic protons.

Preferred compounds include those in which n represents 3.

Suitable R substituents include alkyl and alkoxy groups.

The azo dye group Z is preferably a substituted or unsubstituted phenylazo group. Substituents which may be present in the phenyl group include nitro. Specific examples of suitable azo dye groups include 4-nitrophenylazo and 2,4-dinitrophenylazo.

The ion-sensitive dyes of the invention may be employed to sense cations with which they form a complex. The spectral properties of the dyes make them particularly suitable for use in an optical ion sensor. For example, the sensor may be an optical fibre sensor of the type described in the prior art discussed above in which the ion-sensitive compound is immobilised on the tip of the fibre.

The invention provides a method for the determination of a cation in solution comprising contacting a sample of the solution with a sensor comprising an ion-sensitive compound of the invention to form a complex of the cation with the compound and determining the detectable change resulting from the formation of the complex.

The detectable change may be determined by measuring the spectral absorbance characteristics of the ion-sensitive compound.

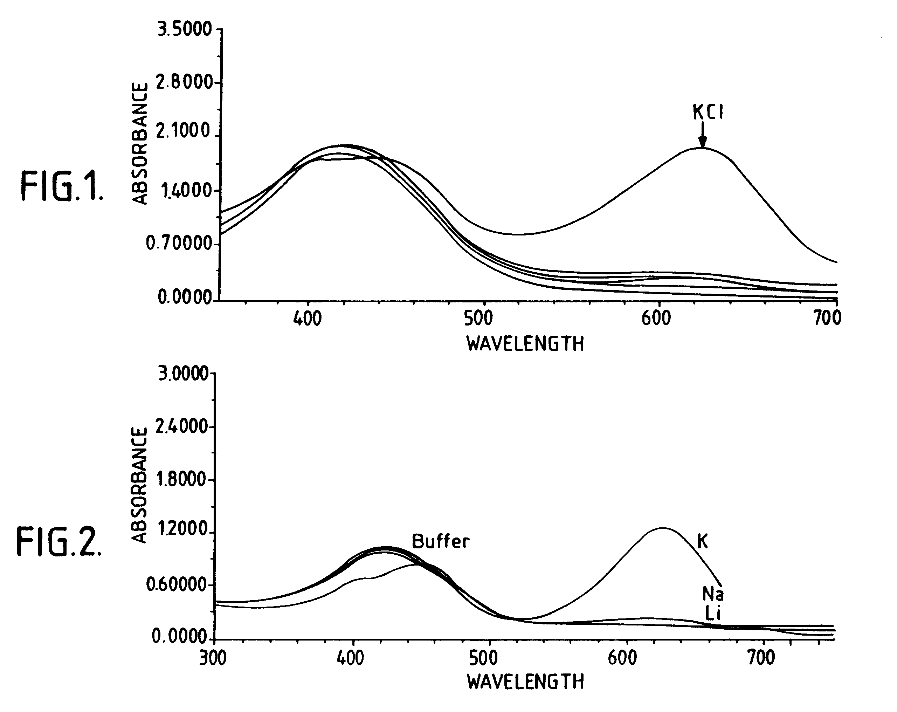

Figures 1 and 2 are graphical representations of spectral absorbance data for compounds of the invention.

The compounds of the invention may be prepared by (i) oxidising a bridged calixarene having the formula 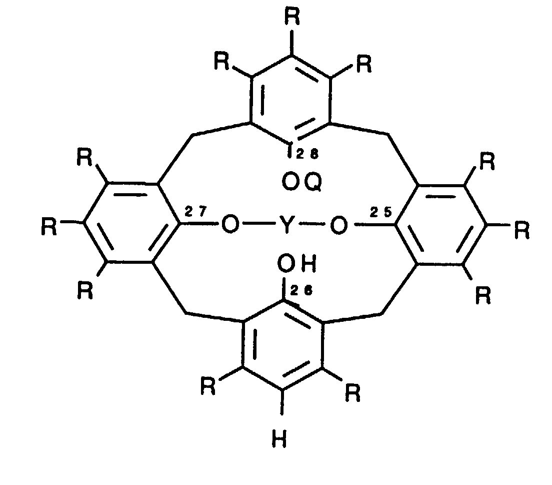 to form the corresponding quinone and (ii) reacting the quinone with an aryl hydrazine of the formula ArNHNH₂ under conditions which result in diazo coupling. In the formulae for the bridged calixarene and the aryl hydrazine, Y, Q, R and Ar are as hereinbefore defined.

Bridged calixarene starting materials of the type having the formula shown have been described in the prior art. For example, representative compounds which are reported to be efficient ionophores for potassium ions are described in J.Am.Chem.Soc., Vol.109, No.15, 1987, 4761-4762.

The oxidation of the bridged calixarene may be performed using thallium trinitrate.

The diazo coupling reaction reaction can be performed in strongly acidic solution, e.g. using concentrated sulphuric acid.

Examples of the preparation and testing of compounds of the invention are as follows.

### Example 1

The preparation of a mono azo dye is outlined in the following reaction scheme and is described in detail thereafter.

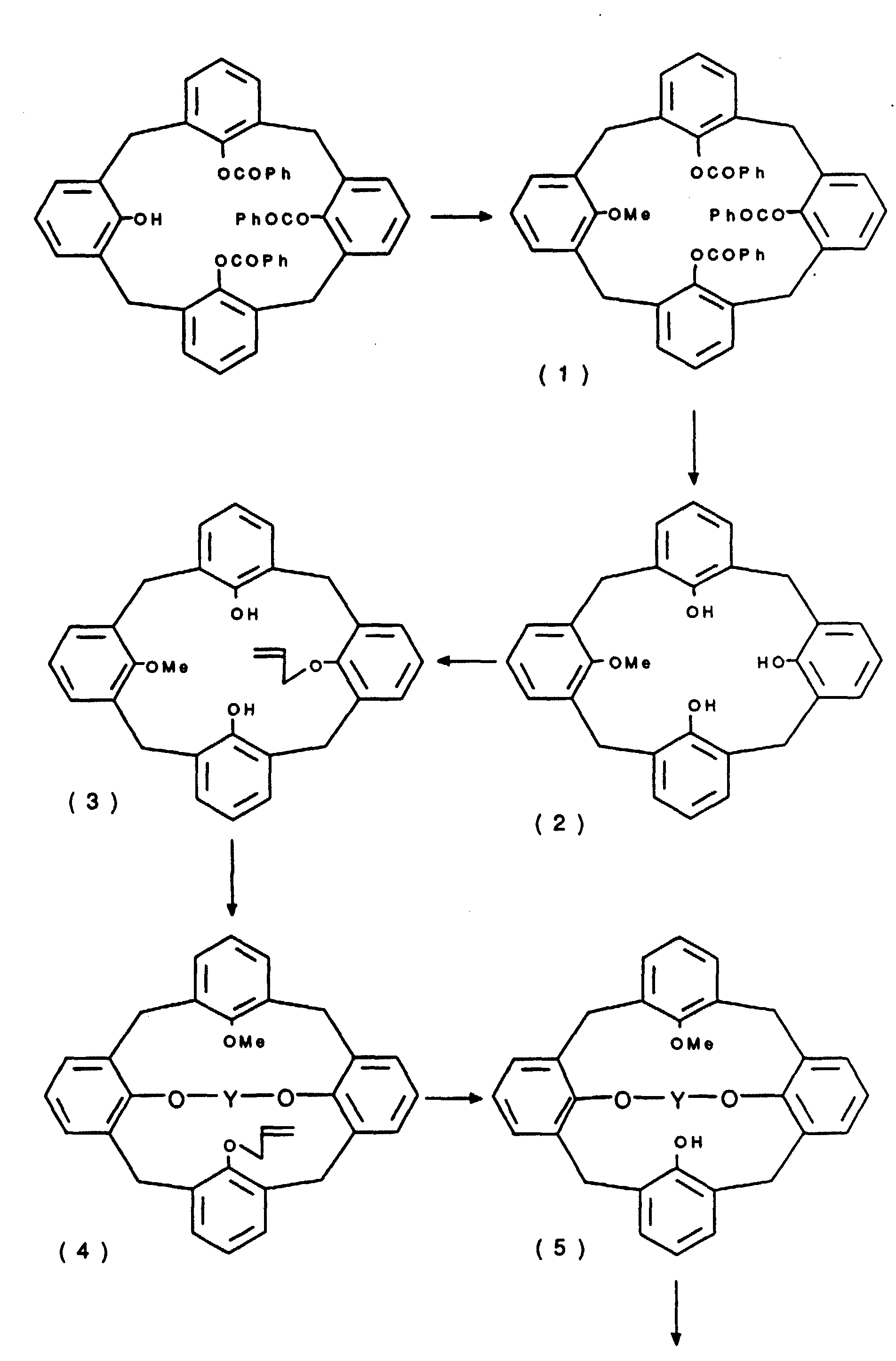

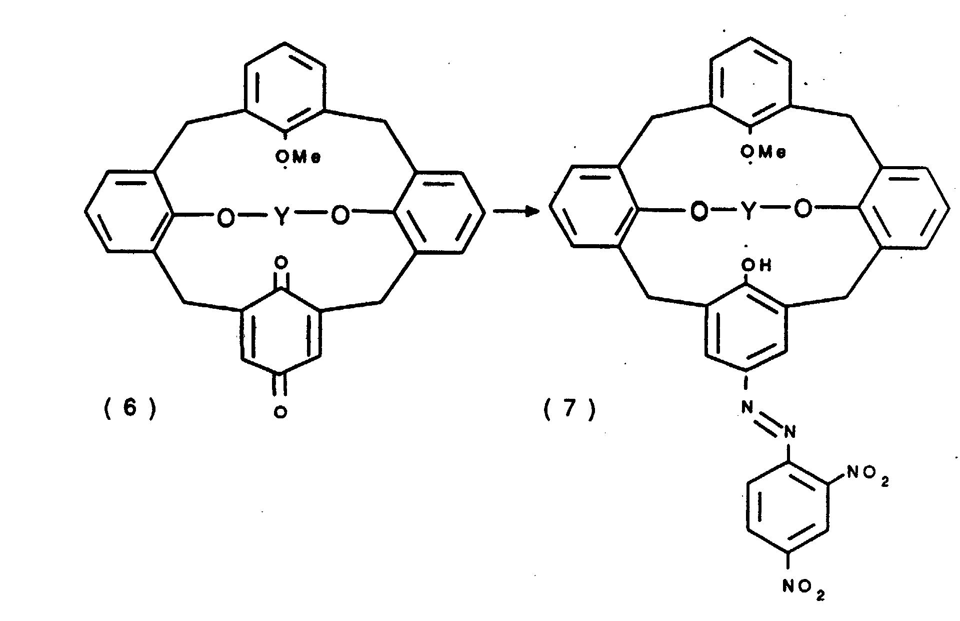

In structures (4)-(7) above, Y represents - (CH₂)₂[O(CH₂)₂]₃-.

### Experimental

### 25,26,27-Tribenzoyloxy-28-methoxycalix[4]arene (1)

A solution of 25,26,27-tribenzoyloxy-28-hydroxycalix[4]arene (14.72g, 20 mmol), sodium hydride (1.92g, 80 mmol, and methyl iodide (7.47ml, 120 mmol) were refluxed in dry THF (400ml) overnight. The solvent was removed by evaporation and the compound triturated with methanol and filtered to give a white powder. Recrystallisation from dichloromethane/methanol gave the required compound (11.2g, 74,6%) as white crystals (m.p. 267-269°C)
- δH(CDCl₃): 8.02-7.13 (m,16,ArH), 6.89-6.43 (m,11,ArH), 3.97-3.34 (m, 8H,ArCH₂Ar) 3.65(s,3,OCH₃)
- Found:: C,76.95; H,4.77 (C₅₀H₃₈O₇.1/2CH₂Cl₂ requires C,77.21; H,5.08)
- M⁺ found:: 750.261 (C₅₀H₃₈O₇ requires 750.262)

### 25,26,27-trihydroxy-28-methoxycalix[4]arene (2)

A solution containing 25,26,27-tribenzoyloxy-28-methoxycalix[4]arene (11.2g, 15 mmol) in THF (400ml) was treated with a solution of sodium hydroxide (35.8g) in water (120ml) and ethanol (280ml), and the mixture refluxed for 18hrs. The solvent was removed by evaporation, the residue was treated with dilute hydrochloric acid and the organic material was extracted into CH₂Cl₂. Evaporation of the solvent gave the crude product which was purified by column chromatography (silica, 50/50 petroleum ether (60/80) chloroform) and recrystallisation from chloroform/methanol gave the product as white crystals (5.62g, 85.9%), m.p. 249-250°C.
- δ(CDCl₃): 9.67 (s,1,ArOH),9.31 (s,2,ArOH) 7.14-6.80(m, 9,ArH),6.67 (t,3,ArH), 4.31 (pair d,4,ArCH₂Ar), 4.07(s,3,OCH₃),3.45 (d,4,ArCH₂Ar).
- Found:: C,79.47 ; H,5.94 (C₂₉H₂₆O₄ requires C, 79.43 ; H,5.98)
- M.S.: M/e 438(M⁺) 100%
- M⁺ found: 438.183 (C₂₉H₂₆O₄ requires 438.183)

### 25.27-dihydroxy-26-allyloxy-28-methoxycalix[4]arene (3)

The calixarene (2) (0.85g, 1.94mmol) and anhydrous potassium carbonate (0.88g,6.38mmol) were mixed in dimethyl ketone and the reaction mixture heated under reflux for one hour. Allyl bromide (0.24g, 1.9mmol) was added and the reaction mixture heated under reflux overnight. The reaction was cooled, the solvent evaporated and the residue partitioned between water (50ml) and dichloromethane (100ml). The aqueous layer was further extracted with dichloromethane (2 x 50 ml). The combined extracts were dried (MgSO₄) and the solvent evaporated to leave the product (0.65g, 70%) which was purified by column chromatography (silica, chloroform) to give a white solid m.p. 164°C.
- δ(CDCl₃): 6.96-6.37 (m,12,ArH), 6.07(m,1, C-CH=C), 5.50(d,2H, C=CH₂), 5.22(d, 2H, C=CH₂), 4.40(d,2H,OCH₂C=C), 4.17 (d,4,ArCH₂Ar), 3.65(s,3,OCH₃), 3.24 (d,4,ArCH₂Ar)
- M.S.: m/e 478 (M⁺) 100%
- M⁺ found:: 478.214 (C₃₂H₃₀O₄ requires 478.214)

### 25,27-(13-crown-5)-26-allyloxy-28-methoxycalix[4]arene (4)

The calixarene (3) (0.5g, 1.04mmol) was dissolved in dry toluene (50ml) and the solution added dropwise to a vigorously stirred suspension of tetraethylene glycol ditosylate (0.52g, 1.05mmol) and ^{t}BuOK (0.26g, 2.3mmol) in dry toluene (50ml) that was heated under reflux. After the addition was complete the reaction mixture was refluxed overnight and then cooled and quenched with water. The toluene was evaporated and the residue partioned between water (20ml) and dichloromethane (30ml). The aqueous layer was extracted with two further portions of dichloromethane and the combined extracts dried (MgSO₄) and the solvent evaporated. Purification was carried out using K⁺ loaded column chromatography to give a white solid m.p. 127°C.
- δ(CDCl₃): 7.17-6.56 (m,12,ArH),6.50-5.87 (m, 3, CH=CH₂), 4.40(m,4,ArCH₂Ar),4.24-3.33 (m,18,OCH₂),4.12(s,3,OCH₃), 3.13(m,4,ArCH₂Ar)
- M⁺ found:: 636.310 (C₄₀H₄₄O₇ requires 636.309)

### 25,27-(13-crown-5)-26-hydroxy-28-methoxycalix[4]arene (5)

A mixture of the calixarene (4) 170mg,0.11mmol), 10% palladium/carbon (120mg,0.11mmol) and p-toluenesulphonic acid (42mg,0.22mmol) were stirred in ethanol (40ml), containing dichloromethane (5ml) under argon for four days. Sodium carbonate (0.1g) was added and the mixture filtered through celite, the celite was then washed with ethanol and then dichloromethane. The solvents were evaporated in vacuo and the compound redissolved in dichloromethane, washed with water several times and dried (MgSO₄).
The crude product was significantly purified using cation loaded column chromatography (silica/10%KBr, dichloromethane up to dichloromethane 3% ethanol, then washed with ethanol). The solvents were evaporated and the compound redissolved in dichloromethane (50ml) and washed with HCl (3M, aqueous solution) and water and evaporated to give the product (0.038g, 57.9%) m.p. 188-198°C.
- δ(CDCl₃): 7.72(s,1,ArOH),7.20-6.87 (m,7,ArH), 6.80(d,2,ArH),6.74-6.54(m,3,ArH),4.37 (m,4,ArCH₂Ar)4.27-3.51(m,19,OCH₂, OCH₃) 3.34 (m,4, ArCH₂Ar)
- M.S.: m/e 596 M⁺ (100%)
- M⁺ found:: 596.277 (C₃₇H₄₀O₇ requires 596.277)

### Mono-quinone (6)

Thallium trinitrate (76mg,0.17mmol) was dissolved in methanol (24ml) and then ethanol (60ml) added. A solution of the calixarene (5) (34mg, 0.05mmol) in chloroform (24ml) was then added, and the resulting yellow solution stirred for ten minutes. On addition of water (60ml) a dark brown mixture resulted which was stirred for a further 15 minutes. The layers were separated and the organic layer washed with water. The solution was evaporated in vacuo and the crude material purified by column chromatography (silica, 3% EtOH:CH₂Cl₂) to give a yellow compound (65mg,83.5%) m.p. 164°C.
- δ(CDCl₃): 7.22-6.28 (m, 9,ArH), 6.07(s,2,quinone H), 4.31-3.1 (m,27,ArCH₂Ar,OCH₂,OCH₃)

### Mono-2,4-dinitroazophenol dye (7)

To a yellow solution of the quinone (50mg,0.08mmol) in chloroform (10ml) was added ethanol (12ml). A solution of 2,4-dinitrophenylhydrazine (5:0g, 25.2 mmol) and concentrated sulphuric acid (10ml) in ethanol (75ml) was prepared. A portion of this (1.2ml,0.4mmol) was added to the solution to give a red solution which was stirred for 1 hour. The solution was evaporated in vacuo and dichloromethane (20ml) added and the solution washed with water and evaporated. The residue was partially purified by prep. chromatography (tlc), the major band was extracted with dichloromethane/methanol. The solvents were evaporated and dichloromethane added (20ml). The solution was washed with HCl (3M, aqueous solution) and water, and evaporated to give the pure mono-azo phenol as dark red crystals (40mg,62%) m.p. 102°C.
δ(CDCl₃, major conformer) ABC system, δ_{A} 8.77, δ_{B} 8.50, δ_{C} 7.88 (J_{AB} 2.4, J_{BC} 8.8 Hz, H-3', H-5' and H-6'), 8.32 (s, OH), 7.82 (s, 2 aryl-H), A₂B system, δ_{A} 7.18, δ_{B} 6.96 (J_{AB} 7.2 Hz, 3 aryl-H), ABC system, δ_{A} 6.71, δ_{B} 6.65, δ_{C} 6.57 (J_{AB}1.2, J_{AC} 7.6, J_{BC 7.6} Hz, 2x3 aryl-H), AB system, δ_{A} 4.60, δ_{B} 3.46 (J_{AB} 13.6 Hz, 2xArCH₂Ar), AB system, δ_{A} 4.36, δ_{B} 3.28 (J_{AB} 12.8 Hz, 2xArCH₂Ar), 4.09 (s, OMe), and 3.7-4.2 (m, 4xOCH₂CH₂O)
δ(CDCl₃, minor conformer) ABC system, δ_{A} 8.78, δ_{B} 8.50, δ_{C} 7.85 (J_{AB} 2.4, J_{BC} 8.8 Hz, H-3', H-5'and H-6'), 8.26 (s, OH), 7.79 (s, 2 aryl-H), A₂B system, δ_{A} 7.27, δ_{B} 7.05 (J_{AB} ca 8 Hz, 3 aryl-H), ABC system, δ_{A} 7.04, δ_{B} 6.91, δ_{C} 6.79 (J_{AB} ca 1, J_{AC} 7.4, J_{BC} 7.4 Hz 2x3 aryl-H), AB system, δ_{A} 4.42, δ_{B} 3.37 (J_{AB} 13.4 Hz, 2xArCH₂Ar).
m/z 790 (M⁺), 791 (M+1)⁺, 813 (M+Na)⁺ and 829 (M+K)⁺.
Found: C, 65.6; H, 5.3; N, 7.0%. C₄₃H₄₂N₄O₁₁ requires C, 65.3; H,5.3; N, 7.1%.

### Solution Experiments

The spectral absorbance characteristics of a 1M solution of the mono-azo dye (7) at pH8 were measured. As shown by the spectral data given in Figure 1, the mono-azo dye shows a modified spectrum in solution when potassium ions are present. Similar modified spectra are not obtained in the presence of other group IA cations such as lithium, sodium, rubidium and caesium as shown in Figure 1. Group IIA cations magnesium and calcium gave no response.

In conclusion, the mono-azo dye is a chromoionophore which is highly selective for potassium ions. A K⁺/Na⁺ discrimination of ca 1000:1 has been demonstrated.

### Example 2

### Mono-A-nitroazophenol dye

In a manner analogous to the preparation of dye (7), a mono-4-nitroazophenol dye was prepared by reacting the quinone (6) with 4-nitrophenylhydrazine.

In solution experiments as described above the mono-4-nitroazophenol dye gave similar results to those achieved for the dye (7) with regard to sensitivity and selectivity.

## Claims

1. A bridged calixorene represented by the formula 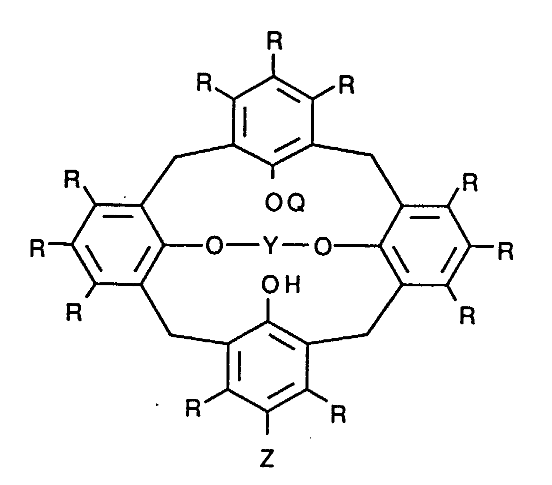 wherein
Y is -(CH₂)₂[O(CH₂)₂]ₙ- in which n is an integer from 1 to 6;
Z is an azo dye group, -N=N-Ar, in which Ar is a substituted or unsubstituted aryl group;
Q is hydrogen or an alkyl group having from 1 to 4 carbon atoms;
each R independently is hydrogen or a substituent free of acidic protons.

2. A compound according to claim 1 wherein n is 3.

3. A compound according to claim 1 or claim 2 wherein each R independently is hydrogen.

4. A compound according to any one of the preceding claims wherein Z is a substituted or unsubstituted phenylazo group.

5. A compound according to claim 4 wherein Z is a 4-nitrophenylazo or a 2,4-dinitrophenylazo group.

6. An optical ion sensor comprising an ion-sensitive compound characterised in that the compound is a bridged calixorene according to any one of the preceding claims.

7. A sensor according to claim 6 wherein the ion-sensitive compound is immobilised on the tip of an optical fibre.

8. A method for the determination of a cation in solution comprising contacting a sample of the solution with a sensor comprising an ion-sensitive compound to form a complex of the cation with the compound and determining the detectable change resulting from the formation of the complex characterised in that the sensor is a sensor according to claim 6 or claim 7.

## Patentansprüche

1. Ein überbrücktes Calixaren, das dargestellt wird durch die Formel 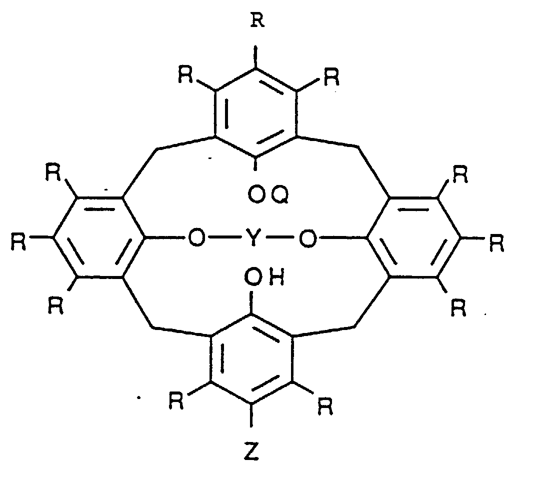 in der
Y -(CH₂)₂[O(CH₂)₂]ₙ ist, worin n eine ganze Zahl von 1 bis 6 ist;
Z eine Azofarbstoffgruppe -N=N-Ar ist, worin Ar eine substituierte oder unsubstituierte Arylgruppe ist;
Q Wasserstoff oder eine Alkylgruppe mit von 1 bis 4 Kohlenstoffatomen ist;
jedes R unabhängig voneinander Wasserstoff oder ein von sauren Protonen freier Substituent ist.

2. Eine Verbindung nach Anspruch 1, dadurch gekennzeichnet, daß n 3 ist.

3. Eine Verbindung nach Anspruch 1 oder Anspruch 2, dadurch gekennzeichnet, daß jedes R unabhängig voneinander Wasserstoff ist.

4. Eine Verbindung nach einem der vorangehenden Ansprüche, dadurch gekennzeichnet, daß Z eine substituierte oder unsubstituierte Phenylazogruppe ist.

5. Eine Verbindung nach Anspruch 4, dadurch gekennzeichnet, daß Z eine 4-Nitrophenylazo- oder eine 2,4-Dinitrophenylazogruppe ist.

6. Ein optischer Ionen-Sensor, der eine ionensensitive Verbindung umfaßt, dadurch gekennzeichnet, daß die Verbindung ein überbrücktes Calixaren nach einem der vorangehenden Ansprüche ist.

7. Ein Sensor nach Anspruch 6, dadurch gekennzeichnet, daß die ionensensitive Verbindung auf der Spitze eines Lichtleiters immobilisiert ist.

8. Ein Verfahren zur Bestimmung eines Kations in Lösung, welches das In-Kontakt-Bringen einer Probe der Lösung mit einem Sensor, der eine ionensensitive Verbindung umfaßt, die einen Komplex des Kations mit der Verbindung bildet, und das Bestimmen der nachweisbaren Veränderung, die aus der Bildung des Komplexes resultiert, umfaßt, dadurch gekennzeichnet, daß der Sensor ein Sensor nach Anspruch 6 oder Anspruch 7 ist.

## Revendications

1. Calixorène ponté représenté par la formule 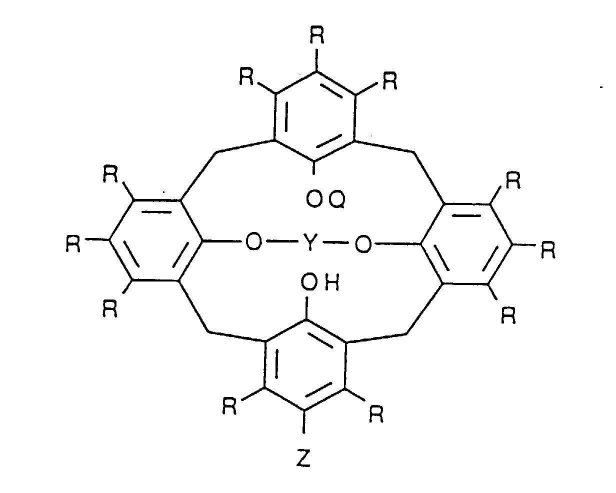 dans laquelle
Y représente -(CH₂)₂[O(CH₂)₂]ₙ-, dans lequel n est un nombre entier de 1 à 6;
Z représente un groupe colorant azoïque, -N=N-Ar, dans lequel Ar représente un groupe aryle substitué ou non substitué ;
Q représente un atome d'hydrogène ou un groupe alkyle contenant de 1 à 4 atomes de carbone ;
chaque R représente indépendamment un atome d'hydrogène ou un substituant sans protons acides.

2. Composé selon la revendication 1, dans lequel n vaut 3.

3. Composé selon la revendication 1 ou la revendication 2, dans lequel chaque R représente indépendamment un atome d'hydrogène.

4. Composé selon l'une quelconque des revendications précédentes, dans lequel Z représente un groupe phénylazo substitué ou non substitué.

5. Composé selon la revendication 4, dans lequel Z représente un groupe 4-nitrophénylazo ou 2,4-dinitrophénylazo.

6. Détecteur d'ions optique, comprenant un composé sensible aux ions, caractérisé en ce que le composé est un calixarène ponté selon l'une quelconque des revendications précédentes.

7. Détecteur selon la revendication 6, dans lequel le composé sensible aux ions est immobilisé à l'extrémité d'une fibre optique.

8. Méthode de détermination d'un cation en solution, comprenant les étapes consistant à mettre en contact un échantillon de la solution avec un détecteur comprenant un composé sensible aux ions pour former un complexe du cation avec le composé et à déterminer la modification détectable due à la formation du complexe, caractérisée en ce que le détecteur est un détecteur selon la revendication 6 ou la revendication 7.
